# EUROPEAN PATENT APPLICATION

(11) **EP 4 483 808 A1**
(43) Date of publication of application: **01.01.2025**
(21) Application number: 23799530.3
(22) Date of filing: 20.02.2023
(51) Int. Cl.: A61B 5/346, A61B 5/00, A61B 5/349, G16H 50/20, G06N 3/08

(54) **METHOD AND DEVICE FOR ANALYZING ELECTROCARDIOGRAM DATA**

(30) Priority: 04.05.2022 KR 20220055152
(71) Applicant: VUNO INC., Seoul 06541 (KR)
(72) Inventor: JOO, Sunghoon, Seoul 04778 (KR); CHANG, Mineok, Seoul 06655 (KR); KIM, Kyung Geun, Seoul 06288 (KR); LEE, Sungjae, Seoul 04582 (KR); NA, Yeongyeon, Seoul 08026 (KR)
(74) Representative: Mewburn Ellis LLP
(86) International application number: PCT/KR2023/002379
(87) International publication number: WO 2023/214647

(57) **Abstract**

Disclosed is a method, performed by a computing device, for analyzing electrocardiogram (ECG) data according to an embodiment of the present disclosure. The method may comprise the steps of: using a first model that uses, as input, ECG data acquired from an ECG measurement device, and determining whether an event related to at least one of atrial fibrillation or atrial flutter is found in the acquired ECG data; generating a first diagnosis result for the acquired ECG data when the event is found in the acquired ECG data; and when the event is not found in the acquired ECG data, generating a second diagnosis result estimating the risk level for at least one of atrial fibrillation or atrial flutter by using a second model that uses the acquired ECG data as input.

## Description

### [Technical Field]

The present disclosure relates to a processing technique of medical data, and more particularly, to a technique for analyzing electrocardiogram data.

### [Background Art]

A waveform of electrocardiogram (ECG), which reflects an electrical activation stage of the heart, is basically composed of detailed regions of a P-wave, QRS-complex, and T-wave, the P wave occurs at the time of atrial depolarization, the QRS complex reflects the time of ventricular depolarization, and the T wave reflects the time of ventricular repolarization.

ECG analysis may refer to a signal processing process that separates the above-described detailed regions from ECG data. The ECG analysis may involve obtaining information about relative time intervals, such as of an amplitude, a PR-interval, an ST-interval, and/or a QT-interval of the detailed regions.

The heart consists of two atria and ventricles. In the heart, an electrical stimulus are generated by electrical cells that have the ability to beat on their own. As such a stimulus is transmitted to the heart muscle cells and the atria and ventricles repeat regular contraction and relaxation, the necessary blood is supplied from the atria to the ventricles and from the ventricles to each organ and tissue.

Atrial fibrillation is a form of tachycardia that occurs in the atrium among arrhythmias. As abnormal electrical signals are conducted into the atrium or abnormal electrical signals are generated within the atrium itself, irregular electrical signals are generated rapidly (for example, at a rate of about 600 times per minute) within the atrium. Because these abnormal electrical signals are conducted rapidly and irregularly, causing the atria to be unable to contract normally (e.g., regularly), the fast electrical signals within the atria may be conducted irregularly to the ventricles through the atrioventricular node. The atrial fibrillation is characterized by a fast heartbeat and irregular intervals between beats. Organic heart diseases such as valvular disease such as mitral valve disease, coronary artery disease, hypertensive heart disease, hypertrophic or dilated cardiomyopathy (heart failure), and congenital heart disease may accompany the atrial fibrillation.

Atrial flutter is a form of tachycardia that occurs in the atria, similar to the atrial fibrillation. The atrial flutter is characterized by regular, abnormal heartbeats that begin suddenly with a rapid heart rate. As such, the atrial flutter is characterized by a relatively slow atrium (e.g., an atrium of approximately 250 to 350 beats per minute) and regularity compared to the atrial fibrillation.

In the early stages of the atrial fibrillation or atrial flutter, the atrial fibrillation or atrial flutter occurs intermittently, so it is difficult to accurately detect an event in which the atrial fibrillation or atrial flutter occurs with a single electrocardiogram (ECG) measurement in a hospital. Long-term ECG measurement, such as a Holter or patch, may be considered, but since the Holter or patch requires continuous ECG measurement for as short as an entire day or as long as two weeks, there is a problem in that it is cumbersome due to a process of attaching electrodes to the skin for a long period of time for diagnosis.

US Patent Publication No. US2021-0076960 discloses ECG based future atrial fibrillation predictor systems and methods.

### [Disclosure]

### [Technical Problem]

The present disclosure is contrived in response to the above-described background art, and has been made in an effort to provide a possibility that atrial fibrillation or atrial flutter event will occur in the future or have occurred in the past by using ECG data in which no atrial fibrillation or atrial flutter event occurs.

### [Technical Solution]

According to an embodiment of the present disclosure for implementing the object, a method for analyzing electrocardiogram (ECG) data performed by a computing device is disclosed. The method may include: determining, by using a first model having ECG data acquired by an ECG measurement device as an input, whether at least one of atrial fibrillation or atrial flutter is found in the acquired ECG data; generating a first diagnosis result for the acquired ECG data when at least one of atrial fibrillation or the atrial flutter is found in the acquired ECG data; and generating a second diagnosis result for estimating a risk for at least one of the atrial fibrillation or the atrial flutter by using a second model having the acquired ECG data as the input when at least one of atrial fibrillation or atrial flutter is not found in the acquired ECG data.

In an embodiment, the first model and the second model may be trained based on different training data, and trained based on different training methods.

In an embodiment, the first model may include at least one of an artificial intelligence-based model trained based on ECG data including at least one of the atrial fibrillation or the atrial flutter, or a rule-based model generated based on feature information for at least one of the atrial fibrillation or the atrial flutter.

In an embodiment, the second model may be an artificial intelligence-based model trained based on normal ECG data in which the atrial fibrillation or the atrial flutter does not occur.

In an embodiment, the second model may be an artificial intelligence-based model trained based on first training data in which first normal ECG data is labeled with a first class representing a high-risk group, and second training data in which second normal ECG data is labeled with a second class representing a low-risk group. The normal ECG data in which abnormal ECG data including at least one of the atrial fibrillation or the atrial flutter within a predetermined time period from an acquisition time point of the normal ECG data exists may be determined as the first normal ECG data.

In an embodiment, the second model is pre-trained based on distinguishing the normal ECG data in which the atrial fibrillation or the atrial flutter does not occur and the abnormal ECG data in which at least one of the atrial fibrillation or the atrial flutter occurs in a dataset with a plurality of ECG data acquired for a first subject, and temporally aligned the distinguished normal ECG data and abnormal ECG data, and generating, based on time information corresponding to each of the distinguished normal ECG data and abnormal ECG data, training data to which a label corresponding to the first class representing the high-risk group or the second class representing the low-risk group is allocated with respect to the normal ECG data.

In an embodiment, the generating of the training data may include generating, additionally based on at least one of prescription record information or surgery history information allocated to the distinguished normal ECG data, the training data to which the label corresponding to the first class representing the high-risk group or the second class representing the low-risk group is allocated with respect to the normal ECG data.

In an embodiment, the generating of the training data may include determining a first time point corresponding to latest ECG data among the plurality of ECG data when the abnormal ECG data does not exist in the plurality of ECG data acquired for the first subject, and generating the training data in which at least one ECG data acquired at time points prior to a first predetermined time period from the first time point is labeled with the second class representing the low-risk group.

In an embodiment, the generating of the training data may include determining a second time point corresponding to first abnormal ECG data among the plurality of ECG data when the normal ECG data and the abnormal ECG data exist in the plurality of ECG data acquired for the first subject, determining a third time point corresponding latest ECG data in at least one ECG data acquired at time points prior to a second predetermined time period from the second time point, and generating the training data in which at least one ECG data acquired at time points prior to a third predetermined time period from the third time point is labeled with the second class representing the low-risk group.

In an embodiment, the second model may analyze a structural change in the atria from the normal ECG data in which the atrial fibrillation or atrial flutter does not occur to output the risk including a possibility of past or future occurrence of the atrial fibrillation or atrial flutter.

In an embodiment, the first diagnosis result may include a result representing that there is a possibility that at least one of the atrial fibrillation or atrial flutter will exist with respect to acquired electrocardiogram data, and the second diagnosis result may include a result representing that at least one of the atrial fibrillation or atrial flutter does not exist with respect to the acquired electrocardiogram data, but there is a possibility that at least one of the atrial fibrillation or atrial flutter will occur with respect to the acquired electrocardiogram data in the past or in the future.

In an embodiment, the second model may include a deep learned based first sub-model which is trained based on a plurality of acquired normal ECG data aligned in a time order and outputs a risk for at least one of the atrial fibrillation or the atrial flutter in response to the acquired ECG data.

In an embodiment, the second model may further include a second sub-model which estimates the heart age from the acquired ECG data and compares the estimated heart age with an actual age of a subject to output the risk for at least one of the atrial fibrillation or the atrial flutter.

In an embodiment, the second model may further include a third sub-model which changes a first threshold used in the first model, which determines whether at least one of the atrial fibrillation or the atrial flutter is found in the acquired ECG data to a second threshold.. Here, the second threshold may be lower than the first threshold, and the third sub-model may output the risk for at least one of the atrial fibrillation and the atrial flutter from the acquired ECG data.

In an embodiment, the second model may further include a fourth sub-model generated based on analyzing ECG characteristic indicators including at least one of a PR-interval, an RR-interval, QRS duration, a QT interval, or a QT-corrected interval for high-risk group ECG data and low-risk group ECG data. The fourth sub-model may output the risk for at least one of the atrial fibrillation and the atrial flutter from the acquired ECG data.

In an embodiment, a computing device for analyzing ECG data is disclosed. The computing device may include: at least one processor; and a memory. The at least one processor may determine, by using a first model having ECG data acquired by ECG measurement device as an input, whether at least one of atrial fibrillation or atrial flutter is found in the acquired ECG data, generate a first diagnosis result for the acquired ECG data when at least one of the atrial fibrillation or atrial flutter is found in the acquired ECG data, and generate a second diagnosis result for estimating a risk for at least one of the atrial fibrillation or atrial flutter by using a second model having the acquired ECG data as the input when at least one of the atrial fibrillation or atrial flutter is not found in the acquired ECG data.

In an embodiment, a computer program stored in a computer readable storage medium is disclosed. When the computer program is executed by at least one processor, the computer program may allow the at least one processor to perform a method for analyzing ECG data, and the method may include determining, by using a first model having ECG data acquired by ECG measurement device as an input, whether at least one of atrial fibrillation or atrial flutter is found in the acquired ECG data, generating a first diagnosis result for the acquired ECG data when the at least one of atrial fibrillation or atrial flutter is found in the acquired ECG data; and generating a second diagnosis result for estimating a risk for at least one of the atrial fibrillation or atrial flutter by using a second model having the acquired ECG data as the input when the at least one of atrial fibrillation or atrial flutter is not found in the acquired ECG data.

### [Advantageous Effects]

A technique according to an embodiment of the present disclosure can provide a possibility that atrial fibrillation or atrial flutter event will occur in the future or have occurred in the past by using ECG data in which no atrial fibrillation or atrial flutter event occurs.

### [Description of Drawings]

The following accompanying drawings are only some of the embodiment of the present disclosure so as to be used for describing the embodiment of the present disclosure, and in the technical field of the present disclosure, those skilled in the technical field of the present disclosure can obtain other drawings based on the drawings without an effort to reach a new invention.
FIG. 1 is an exemplary block diagram of a computing device for performing an operation for analyzing electrocardiogram data according to an embodiment of the present disclosure.
FIG. 2a exemplarily illustrates a method for analyzing electrocardiogram data according to an embodiment of the present disclosure.
FIG. 2b exemplarily illustrates operations of a first model and a second model that perform the method for analyzing ECG data according to an embodiment of the present disclosure.
FIG. 3 schematically illustrates an exemplary operation scheme of a risk estimation model according to an embodiment of the present disclosure.
FIG. 4 illustrates an exemplary algorithm for building training data for electrocardiogram data according to an embodiment of the present disclosure.
FIG. 5 is a block diagram of a generalized computing device according to an embodiment of the present disclosure.

### [Mode for Invention]

Various exemplary embodiments will now be described with reference to drawings. In the present specification, various descriptions are presented to provide appreciation of the present disclosure. However, it is apparent that the exemplary embodiments can be executed without the specific description.

"Component", "module", "system", and the like which are terms used in the specification refer to a computer-related entity, hardware, firmware, software, and a combination of the software and the hardware, or execution of the software. For example, the component may be a processing procedure executed on a processor, the processor, an object, an execution thread, a program, and/or a computer, but is not limited thereto. For example, both an application executed in a computing device and the computing device may be the components. One or more components may reside within the processor and/or a thread of execution. One component may be localized in one computer. One component may be distributed between two or more computers. Further, the components may be executed by various computer-readable media having various data structures, which are stored therein. The components may perform communication through local and/or remote processing according to a signal (for example, data transmitted from another system through a network such as the Internet through data and/or a signal from one component that interacts with other components in a local system and a distribution system) having one or more data packets, for example.

The term "at least one of A or B" should be interpreted to mean "a case including only A", "a case including only B", and "a case in which A and B are combined".

The term expressed N, such as first, second, or third is used to distinguish at least one entity. For example, entities expressed as first and second may be identical or different from each other depending on the implementation aspect.

The term "or" is intended to mean not exclusive "or" but inclusive "or". That is, when not separately specified or not clear in terms of a context, a sentence "X uses A or B" is intended to mean one of the natural inclusive substitutions. That is, the sentence "X uses A or B" may be applied to any of the case where X uses A, the case where X uses B, or the case where X uses both A and B. Further, it should be understood that the term "and/or" used in this specification designates and includes all available combinations of one or more items among enumerated related items.

It should be appreciated that the term "comprise" and/or "comprising" means presence of corresponding features and/or components. However, it should be appreciated that the term "comprises" and/or "comprising" means that presence or addition of one or more other features, components, and/or a group thereof is not excluded. Further, when not separately specified or it is not clear in terms of the context that a singular form is indicated, it should be construed that the singular form generally means "one or more" in this specification and the claims.

Those skilled in the art need to recognize that various illustrative logical blocks, configurations, modules, circuits, means, logic, and algorithm steps described in connection with the exemplary embodiments disclosed herein may be additionally implemented as electronic hardware, computer software, or combinations of both sides. To clearly illustrate the interchangeability of hardware and software, various illustrative components, blocks, configurations, means, logic, modules, circuits, and steps have been described above generally in terms of their functionalities. Whether the functionalities are implemented as the hardware or software depends on a specific application and design restrictions given to an entire system. Skilled artisans may implement the described functionalities in various ways for each particular application. However, such implementation decisions should not be interpreted as causing a departure from the scope of the present disclosure.

The description of the presented exemplary embodiments is provided so that those skilled in the art of the present disclosure use or implement the present disclosure. Various modifications to the exemplary embodiments will be apparent to those skilled in the art. Generic principles defined herein may be applied to other embodiments without departing from the scope of the present disclosure. Therefore, the present disclosure is not limited to the exemplary embodiments presented herein. The present disclosure should be analyzed within the widest range which is coherent with the principles and new features presented herein.

In the present disclosure, "electrocardiogram data," "ECG," "ECG signal", and "ECG data" may be used interchangeably.

Evidence has been reported that most patients in the early stages of atrial fibrillation or atrial flutter, which occurs immediately before or intermittently, have structural changes in the atria (e.g., cardiomyopathy) that predispose them to atrial arrhythmia (Kottkamp, H. Human atrial fibrillation substrate: Towards a specific fibrotic atrial cardiomyopathy. Eur. Heart J. 34, 2731-2738 (2013)).

Accordingly, although it is difficult to detect the atrial fibrillation or atrial flutter with the naked eye from ECG data measured at this early stage, artificial intelligence or deep learning algorithms that can learn important features from the ECG data may be possible to detect subtle changes that are more likely to progress to the atrial fibrillation or atrial flutter from the ECG data.

According to an embodiment of the present disclosure, a method of presenting the risk that the atrial fibrillation or atrial flutter may occur in the future or in the past is provided by using ECG data in which no event related to atrial fibrillation or atrial flutter occurs. A technique according to an embodiment of the present disclosure can present the possibility that atrial fibrillation or atrial flutter occurs in a past certain period of time (or the possibility of occurrence in the future) from normal ECG data at the current time point, so compared to existing techniques, the range of risk groups for heart-related diseases presented by the algorithm can be expanded more broadly.

As used herein, the expressions "normal electrocardiogram data" or "normal electrocardiogram data" mean electrocardiogram data in which atrial fibrillation or atrial flutter is not found. As a non-limited example, electrocardiogram data in which diseases other than atrial fibrillation or atrial flutter (e.g., heart failure, etc.) are found but no atrial fibrillation or atrial flutter is found, may be also included in the category of the normal electrocardiogram data used herein. As used herein, the expressions "abnormal electrocardiogram data" or "abnormal electrocardiogram data" may mean electrocardiogram data in which the atrial fibrillation or atrial flutter is found.

Considering that it is important for prognosis that treatment of the atrial fibrillation and atrial flutter is performed within 1 year from the time point of first occurrence, the technique according to an embodiment of the present disclosure may be helpful in health management and treatment for patients just before the occurrence of the atrial fibrillation or atrial flutter or patients in whom it is difficult to measure events related to the atrial fibrillation or atrial flutter through electrocardiogram measurement due to intermittent events occurring in the early stages of the atrial fibrillation or atrial flutter.

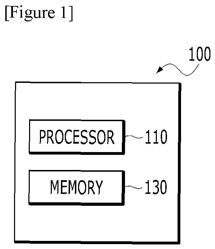

FIG. 1 is a diagram illustrating a block diagram of a computing device 100 for analyzing electrocardiogram data according to an embodiment of the present disclosure.

A configuration of the computing device 100 illustrated in FIG. 1 is only an example shown through simplification. In an embodiment of the present disclosure, the computing device 100 may include other components for performing a computing environment of the computing device 100, and only some of the disclosed components may also constitute the computing device 100.

The computing device 100 according to an embodiment of the present disclosure may include a processor 110 and a memory 130.

A configuration of the computing device 100 illustrated in FIG. 1 is only an example shown through simplification. In an embodiment of the present disclosure, the computing device 100 may include other components for performing the computing environment of the computing device 100, and only some of the disclosed components may constitute the computing device 100.

In the present disclosure, the computing device 100 may mean a node constituting a system for implementing embodiments of the present disclosure. The computing device 100 may mean any type of user terminal or any type of server. The components of the computing device 100 may be exemplary and some components may be excluded, or an additional component may be included in the computing device 100. As an example, when the computing device 100 includes a terminal, an output unit (not illustrated) and an input unit (not illustrated) may be included in a range of the computing device 100.

The computing device 100 in the present disclosure may perform technical features according to embodiments of the present disclosure to be described below. For example, the computing device 100 may determine whether an event related to at least one of the atrial fibrillation or atrial flutter found in the acquired electrocardiogram data by using a first model using electrocardiogram data acquired by electrocardiogram measurement as an input. The computing device 100 may generate a first diagnosis result for the acquired electrocardiogram data when the event is found in the acquired electrocardiogram data, and generate a second diagnosis result of estimating a risk for at least one of the atrial fibrillation or the atrial flutter by using a second model using the acquired electrocardiogram data as the input when the event is not found in the acquired electrocardiogram data.

In an embodiment, the processor 110 may be constituted by at least one core and may include processors for data analysis and/or processing, which include a central processing unit (CPU), a general purpose graphics processing unit (GPGPU), a tensor processing unit (TPU), and the like of the computing device 100.

The processor 110 may read a computer program stored in the memory 130 and perform operations according to an embodiment of the present disclosure. For example, the processor 110 may perform operations for generating a first diagnosis result representing that there is a possibility that at least one of the atrial fibrillation or atrial flutter will exist with respect to acquired electrocardiogram data, and/or a second diagnosis result representing that at least one of the atrial fibrillation or atrial flutter does not exist with respect to the acquired electrocardiogram data, but there is a possibility that at least one of the atrial fibrillation or atrial flutter will occur with respect to the acquired electrocardiogram data in the past or in the future.

According to an additional embodiment of the present disclosure, the processor 110 may also perform a computation for learning a neural network. The processor 110 may perform calculations for learning the neural network, which include processing of input data for learning in deep learning (DL), extracting a feature in the input data, calculating an error, updating a weight of the neural network using backpropagation, and the like. At least one of the CPU, GPGPU, and TPU of the processor 110 may process learning of a network function. For example, both the CPU and the GPGPU may process the learning of the network function and data classification using the network function. As an example, the processor 110 may perform an operation of training a first model for determining whether an event related to the atrial fibrillation or atrial flutter is found in the ECG data. As another example, the processor 110 may perform an operation of training a second model for estimating a risk related to the atrial fibrillation or atrial flutter is found in the ECG data. The first model and/or the second model in the present disclosure may be used to refer to a computer-related entity, hardware, firmware, software, and a combination of the software and the hardware, or execution of the software.

Further, in an embodiment of the present disclosure, processors of the plurality of computing devices may be used together to perform the learning of the network function and the data processing using the network function. Further, the computer program executed in the computing device according to an embodiment of the present disclosure may be a CPU, GPGPU, or TPU executable program.

Additionally, the processor 110 may generally process an overall operation of the computing device 100. For example, the processor 110 processes data, information, signals, and the like input or output through the components included in the computing device 100 or drives the application program stored in a storage unit to provide or process information or a function appropriate for the user.

According to an embodiment of the present disclosure, the memory 130 may store any type of information generated or determined by the processor 110 or any type of information received by the computing device 100. According to an embodiment of the present disclosure, the memory 130 may be a storage medium that stores computer software which allows the processor 110 to perform the operations according to the embodiments of the present disclosure. Therefore, the memory 130 may mean computer-readable media for storing software codes required for performing the embodiments of the present disclosure, data which become execution targets of the codes, and execution results of the codes.

According to an embodiment of the present disclosure, the memory 130 may mean any type of storage medium, and include, for example, at least one type of storage medium of a flash memory type storage medium, a hard disk type storage medium, a multimedia card micro type storage medium, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. The computing device 100 may operate in connection with a web storage performing a storing function of the memory 130 on the Internet. The description of the memory is just an example and the memory 130 used in the present disclosure is not limited to the examples.

In the present disclosure, the communication unit (not illustrated) may be configured regardless of communication modes such as wired and wireless modes and constituted by various communication networks including a personal area network (PAN), a wide area network (WAN), and the like. Further, the network unit 150 may operate based on known World Wide Web (WWW) and may adopt a wireless transmission technology used for short-distance communication, such as infrared data association (IrDA) or Bluetooth.

The computing device 100 in the present disclosure may include any type of user terminal and/or any type of server. Therefore, the embodiments of the present disclosure may be performed by the server and/or the user terminal.

The user terminal may include any type of terminal which is capable of interacting with the server or another computing device. The user terminal may include, for example, a mobile phone, a smart phone, a laptop computer, personal digital assistants (PDA), a slate PC, a tablet PC, and an Ultrabook.

The server may include, for example, any type of computer system or computing device such as a microprocessor, a mainframe computer, a digital processor, a portable device, and a device controller.

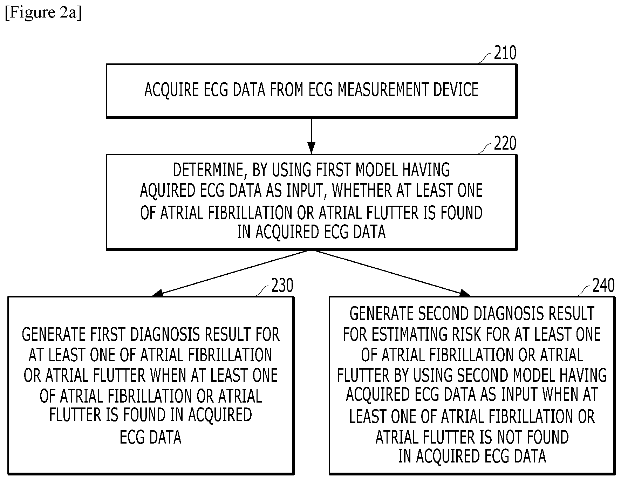

FIG. 2a exemplarily illustrates a method for analyzing electrocardiogram data according to an embodiment of the present disclosure.

The method illustrated in FIG. 2a may be performed by the computing device 100, for example. The steps illustrated in FIG. 2a are exemplarily expressed according to an embodiment of the present disclosure, and it will be apparent to those skilled in the art that additional steps may exist, some steps may be omitted, or some steps may be replaced with other steps.

The computing device 100 may acquire electrocardiogram data from an electrocardiogram measurement device (210).

In an embodiment of the present disclosure, the electrocardiogram data may refer to any type of signal or information representing an electrical activity of the heart.

Additionally, the electrocardiogram data in the present disclosure may be used as an example for a biological signal. Additionally, the electrocardiogram data in the present disclosure may be used as an example for a heart-related signal.

In an embodiment of the present disclosure, the electrocardiogram measurement device may refer to any type of device that may measure an electrical signal of the heart that represents a potential change shown in relation to heart beat. For example, the electrocardiogram measurement device may detect and amplify a minute electrical signal detected on the skin when the myocardium depolarizes with each heartbeat.

For example, the electrocardiogram measurement device may be a device with N leads (where N is a natural number). In such an example, the electrocardiogram measurement device may be operated in a scheme such as 3-lead, 5-lead, or 12-lead, for example. As another example, the electrocardiogram measurement device may have various forms, such as a wearable form or a patch form. As yet another example, the electrocardiogram measurement device may include the computing device 100. In such an example, the computing device 100 itself may measure the electrocardiogram and analyze a result thereof. In this way, the ECG data may refer to data acquired using electrodes of the ECG measurement device which exists outside the computing device, or may refer to data received from electrodes directly connected to the computing device 100.

The computing device 100 may determine whether at least one of the atrial fibrillation or the atrial flutter is found in the acquired electrocardiogram data by using the first model using the acquired electrocardiogram data as the input (S220).

In an embodiment of the present disclosure, the expression "atrial fibrillation or atrial flutter" herein may be used as an example of expressions such as abnormal heart rate rhythm, heart disease, and/or abnormal heart rate. Accordingly, it will be clearly understood by those skilled in the art that embodiments of the present disclosure for generating diagnosis results related to the atrial fibrillation or atrial flutter may also be applied to abnormalities in heart rate rhythm, heart disease, and/or abnormal heart beat. Therefore, for convenience of explanation, the atrial fibrillation or atrial flutter will be used hereinafter as representative examples of the abnormal heart beat rhythm, heart disease, and/or abnormal heart rate. In this way, the heart beat rhythm abnormalities, heart disease, and/or abnormal heart beat, and the atrial fibrillation or atrial flutter may be used interchangeably.

In an embodiment of the present disclosure, a first model may be utilized to determine whether at least one of the atrial fibrillation or flutter is found in the ECG data. The first model may include an artificial intelligence-based model trained based on electrocardiogram data including at least one of the atrial fibrillation or the atrial flutter, and/or a rule-based model generated based on feature information for at least one of the atrial fibrillation or atrial flutter.

For example, the first model may mean an algorithm that reads whether the atrial fibrillation or atrial flutter occurs when the ECG data is input. As an example, the algorithm that reads whether the atrial fibrillation or atrial flutter occurs may be a deep learning-based model that learns the ECG data related to the atrial fibrillation or atrial flutter. As another example, an algorithm that reads whether the atrial fibrillation or atrial flutter occurs may be an algorithm that uses features of the ECG data based on a rule.

In an embodiment of the present disclosure, the first model may generate a first diagnosis result indicating that the atrial fibrillation or atrial flutter is found in the currently measured ECG data when the atrial fibrillation or atrial flutter exists in the input ECG data. That is, the computing device 100 may generate a first diagnosis result for at least one of the atrial fibrillation or the atrial flutter when an event related to at least one of the atrial fibrillation or the atrial flutter exists in the acquired ECG data (S230).

For example, the first diagnosis result may include a content representing that the atrial fibrillation or atrial flutter is found in currently measured electrocardiogram data. As an additional example, the first diagnosis result may also include a quantitative value for heart beats per minute along with a result for the atrial fibrillation or atrial flutter and/or a quantitative value including a value representing how severe the atrial fibrillation or atrial flutter is represented. As an additional example, the first diagnosis result may further include exercise information to reduce the atrial fibrillation or atrial flutter, dietary information, and/or hospital information. As an additional example, the first diagnosis result may further include information about drug treatment and/or surgical treatment for treating the atrial fibrillation or atrial flutter.

In an embodiment of the present disclosure, the first model may generate a result indicating that the atrial fibrillation or atrial flutter is not found in the currently measured ECG data when the atrial fibrillation or atrial flutter does not exist in the input ECG data.

In an embodiment of the present disclosure, the computing device 100 may generate a second diagnosis result for estimating a risk for at least one of the atrial fibrillation or atrial flutter by using a second model using the acquired ECG data as the input when the atrial fibrillation or atrial flutter is not found in the acquired ECG data (S240).

The computing device 100 may generate the second diagnosis result for estimating the risk for the atrial fibrillation or atrial flutter from ECG data in which no atrial fibrillation or atrial flutter occurs. For example, the second diagnosis result may include information indicating the possibility of future or past atrial fibrillation or atrial flutter, assuming that the atrial fibrillation or atrial flutter is not found in the currently measured ECG data. As an example, the second diagnosis result may include a quantitative value indicating the risk for the atrial fibrillation or atrial flutter.

As an example, the second model may mean an artificial intelligence-based model learned based on normal ECG data in which no event related to the atrial fibrillation or atrial flutter occurs. Therefore, according to the technique according to an embodiment of the present disclosure, no events related to the atrial fibrillation or atrial flutter is found in the currently measured ECG data, but it is possible to quantitatively show how much the risk for the atrial fibrillation or atrial flutter exists, it may be possible to more accurately and efficiently provide information to a user.

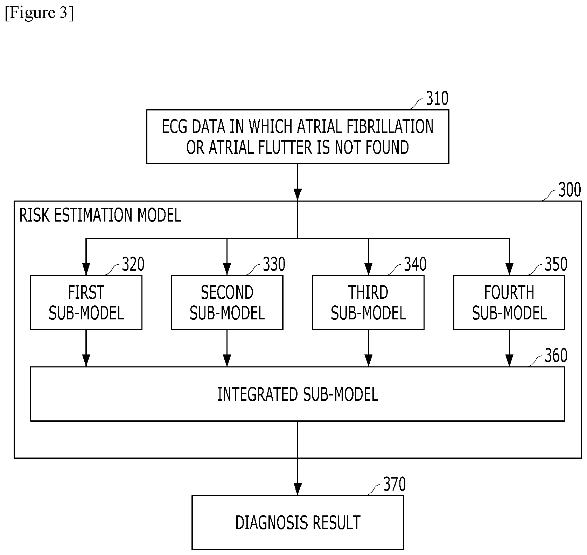

An algorithm for estimating the risk of the atrial fibrillation or atrial flutter from the ECG data in which no atrial fibrillation or atrial flutter occurs will be described later in FIG. 3.

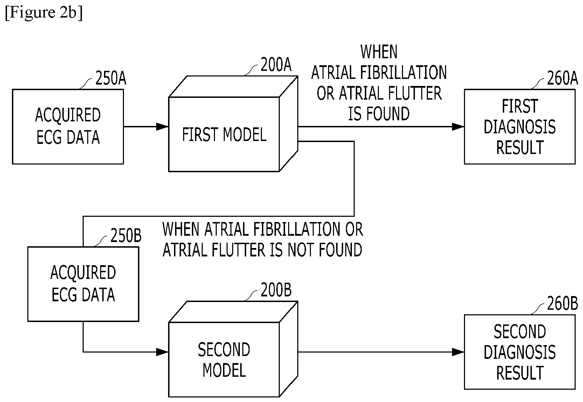

FIG. 2b exemplarily illustrates operations of a first model 200A and a second model 200B that perform the method for analyzing ECG data according to an embodiment of the present disclosure.

For example, the operations illustrated in FIG. 2b may be performed by the computing device 100.

In an embodiment of the present disclosure, the first model 200A may input acquired ECG data 250A, and outputs a first diagnosis result 260A when the atrial fibrillation or atrial flutter is found.

For example, the first diagnosis result 260A may include a content representing that an event for a heart-related disease is found in currently measured electrocardiogram data. The heart-related disease may include a heart rate of an abnormal rhythm. Further, the first diagnosis result 260A may include a content representing that the atrial fibrillation or atrial flutter is found. As an additional example, the first diagnosis result 260A may also include a quantitative value for heart beats per minute along with a result for the atrial fibrillation or atrial flutter and/or a quantitative value including a value representing how severe the atrial fibrillation or atrial flutter is represented. As an additional example, the first diagnosis result 260A may further include exercise information to reduce the atrial fibrillation or atrial flutter, dietary information, and/or hospital information. As an additional example, the first diagnosis result 260A ay further include information about drug treatment and/or surgical treatment for treating the atrial fibrillation or atrial flutter.

In an embodiment, the first model 200A may mean an artificial intelligence-based model learned based on electrocardiogram data including at least one of the atrial fibrillation or the atrial flutter, or a rule-based model generated based on feature information for at least one of the atrial fibrillation or atrial flutter.

When the atrial fibrillation or the atrial flutter is not found in the first model 200A, the acquired ECG data 250B may be delivered to the second model 200B. In an embodiment of the present disclosure, the ECG data 250A and the ECG data 250B may mean the same data. In an additional embodiment of the present disclosure, the ECG data 250A and the ECG data 250B may be different from each other. For example, the ECG data 250B output from the first model 200A may mean data to which additional processing is applied to the ECG data 250A to facilitate processing in the second model 200B. As an example, the ECG data 250B may mean data from which unnecessary information such as noise is removed from the ECG data 250A as a result of analysis in the first model 200A.

In an embodiment of the present disclosure, the second model 200B may generate a second diagnosis result 260B by inputting the ECG data 250B. The second diagnosis result 260B may include a risk for the atrial fibrillation or atrial flutter in ECG data in which no atrial fibrillation or atrial flutter occurs. For example, the second diagnosis result 260B may include information indicating the possibility of future or past atrial fibrillation or atrial flutter, assuming that the atrial fibrillation or atrial flutter is not found in the currently measured ECG data. As an example, the second diagnosis result 260B may include a quantitative value indicating the risk for the atrial fibrillation or atrial flutter.

In an embodiment of the present disclosure, when the first model 200A is an artificial intelligence-based model, the first model 200A and the second model 200B may be trained based on different training data, and trained based on different training methods. For example, the first model 200A may be learned by using ECG data labeled with information about whether the atrial fibrillation or atrial flutter exists as learning data. For example, the second model 200B may be trained to output a result predicting the risk for the atrial fibrillation or atrial flutter from normal ECG data in which the atrial fibrillation or atrial flutter does not exist. When abnormal ECG data (e.g., the ECG data in which atrial fibrillation or atrial flutter occurred) exists within a predetermined time period from normal ECG data for the same subject, the second model 200B may be trained by using training data in which the normal ECG data is labeled with information that the risk is high (e.g., high-risk group labeling).

In an embodiment of the present disclosure, the second model 200B may mean an artificial intelligence-based model trained based on normal ECG data in which no event related to the atrial fibrillation or atrial flutter occurs. The second model 200B analyzes a structural change in the atria from the normal ECG data in which no event related to atrial fibrillation or atrial flutter occurs, thereby outputting the risk including a possibility of past or future occurrence of the atrial fibrillation or atrial flutter.

For example, the second model 200B may mean an artificial intelligence-based model trained based on first training data in which first normal ECG data is labeled with a first class representing a high-risk group, and second training data in which second normal ECG data is labeled with a second class representing a low-risk group. Here, the high-risk group and the low-risk group are class names exemplified for the purpose of explanation, and the class name may be changed to a risk group or a non-risk group depending on an implementation aspect. Further, here, the predetermined period may be set to various periods including one year, sixth months, one month, one week, etc., depending on an implementation aspect. As described above, in the technique according to an embodiment of the present disclosure, information on the risk group may be additionally determined even for the normal ECG data in which no event related to the atrial fibrillation or atrial flutter occurs, so that for the currently measured ECG data, a risk related to the atrial fibrillation or atrial flutter may be presented along with the determination of whether there is the event related to the atrial fibrillation or atrial flutter.

In an embodiment of the present disclosure, during a training process of the second model 200B, the normal ECG data in which no event related to the atrial fibrillation or atrial flutter occurs and the abnormal ECG data in which the event occurs may be distinguished from each other, and temporally aligned in a dataset with a plurality of ECG data acquired with respect to a first subject. ECG data measured multiple times by respective subjects may be temporally aligned, and the plurality of ECG data temporally aligned may be represented differently depending on normal and abnormal cases for the plurality of ECG data temporally aligned. In an embodiment of the present disclosure, based on time information corresponding to each of the distinguished normal ECG data and abnormal ECG data, training data to which a label corresponding to the first class representing the high-risk group or the second class representing the low-risk group is allocated may be generated with respect to the normal ECG data. In an embodiment, the training data used in the second model 200B may be generated by a process of determining whether the normal ECG data is dangerous ECG data using time information of the normal ECG data and time information of the abnormal ECG data. For example, for the same subject, if the abnormal ECG data is acquired at a time point temporally close to a time point when the normal ECG data is acquired, the normal ECG data is not data in which the atrial fibrillation or atrial flutter occurs, but it may be determined that the occurrence possibility of the atrial fibrillation or atrial flutter is relatively high. Therefore, by comparing the acquisition time point of the normal ECG data and the acquisition time point of the abnormal ECG data for the same subject, the end and/or value of the risk for the normal ECG may be determined.

In an additional embodiment of the present disclosure, additional meta information about a subject may be utilized to generate training data. Examples of the meta information may include surgery history information related to a heart disease and/or prescription record information related to the heart disease. Even in the normal electrocardiogram data, when medication prescribed for the heart disease is taken at the relevant time point or at time points near the relevant time point, or when there is the surgery history for the heart disease, the normal ECG data or another normal ECG data temporally adjacent to the normal ECG data may be considered as normal ECG data with a high-risk.

In an additional embodiment of the present disclosure, additional meta information about the subject may be used as information embedded in an inference process of the second model 200B.

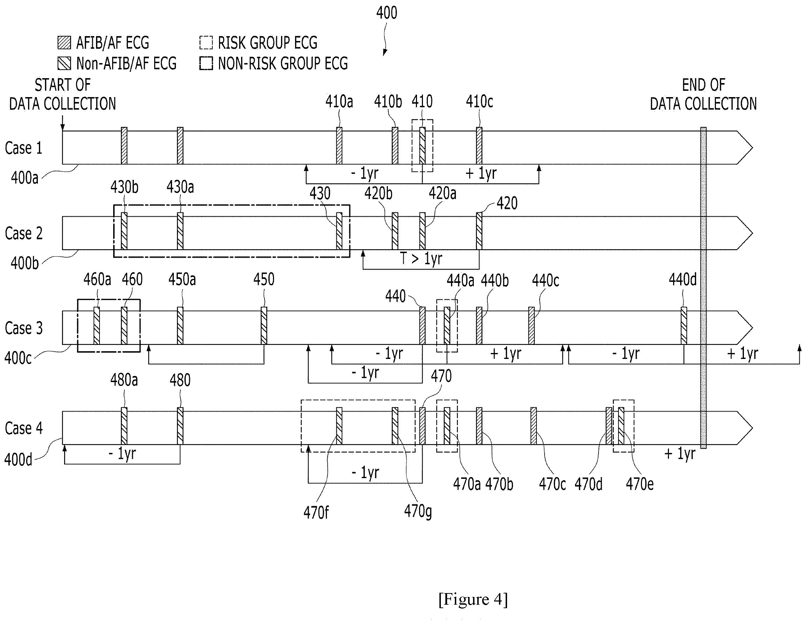

A specific scheme of generating the training data for the second model 200B will be described below in FIG. 4.

FIG. 3 schematically illustrates an exemplary operation scheme of a risk estimation model 300 according to an embodiment of the present disclosure.

In an embodiment, the risk estimation model 300 in FIG. 3 may correspond to the second model 200B. In FIG. 3, an operation (e.g., inference, etc.) process of sub-models of the risk estimation model 300 will be described.

The risk estimation model 300 may receive (normal) ECG data 310 in which atrial fibrillation or atrial flutter is not found. The risk estimation model 300 may include a plurality of sub-models 320, 330, 340, and 350 by inputting the ECG data 310.

In an embodiment, the risk estimation model 300 may generate a diagnosis result 370 using at least one of the plurality of sub-models 320, 330, 340, and 350. Here, the diagnosis result 370 may correspond to the second diagnosis result 260B.

In an additional embodiment, the risk estimation model 300 may further include an integrated sub-model 360 that integrates or ensembles outputs of the plurality of sub-models 320, 330, 340, and 350. The integrated sub-model 360 may select or combine at least one of a plurality of outputs acquired from the plurality of sub-models 320, 330, 340, and 350. Additionally, the integrated sub-model 360 may select or combine at least one of the plurality of outputs acquired from the plurality of sub-models 320, 330, 340, and 350 according to an analysis result of the input ECG data 310. As another example, the integrated sub-model 360 post-processes (e.g., averages, etc.) risk scores included in the plurality of outputs acquired from the plurality of sub-models 320, 330, 340, and 350 to generate a diagnosis result 370.

In an embodiment of the present disclosure, a first sub-model 320 may include a neural network based model that is trained based on a plurality of normal ECG data aligned in order of acquisition time and outputs a risk for at least one of the atrial fibrillation or the atrial flutter in response to the acquired ECG data 310. For example, the neural network-based model may include a deep learning-based model.

Throughout the present specification, a computation model, the neural network, a network function, and the neural network may be used as the same meaning. The neural network may be generally constituted by an aggregate of calculation units which are mutually connected to each other, which may be called nodes. The nodes may also be called neurons. The neural network is configured to include one or more nodes. The nodes (alternatively, neurons) constituting the neural networks may be connected to each other by one or more links.

In the neural network, one or more nodes connected through the link may relatively form the relationship between an input node and an output node. Concepts of the input node and the output node are relative and a predetermined node which has the output node relationship with respect to one node may have the input node relationship in the relationship with another node and vice versa. As described above, the relationship of the input node to the output node may be generated based on the link. One or more output nodes may be connected to one input node through the link and vice versa.

In the relationship of the input node and the output node connected through one link, a value of data of the output node may be determined based on data input in the input node. Here, a link connecting the input node and the output node to each other may have a weight. The weight may be variable and the weight is variable by a user or an algorithm in order for the neural network to perform a desired function. For example, when one or more input nodes are mutually connected to one output node by the respective links, the output node may determine an output node value based on values input in the input nodes connected with the output node and the weights set in the links corresponding to the respective input nodes.

As described above, in the neural network, one or more nodes are connected to each other through one or more links to form a relationship of the input node and output node in the neural network. A characteristic of the neural network may be determined according to the number of nodes, the number of links, correlations between the nodes and the links, and values of the weights granted to the respective links in the neural network. For example, when the same number of nodes and links exist and there are two neural networks in which the weight values of the links are different from each other, it may be recognized that two neural networks are different from each other.

The neural network may be constituted by a set of one or more nodes. A subset of the nodes constituting the neural network may constitute a layer. Some of the nodes constituting the neural network may constitute one layer based on the distances from the initial input node. For example, a set of nodes of which distance from the initial input node is n may constitute n layers. The distance from the initial input node may be defined by the minimum number of links which should be passed through for reaching the corresponding node from the initial input node. However, a definition of the layer is predetermined for description and the order of the layer in the neural network may be defined by a method different from the aforementioned method. For example, the layers of the nodes may be defined by the distance from a final output node.

The initial input node may mean one or more nodes in which data is directly input without passing through the links in the relationships with other nodes among the nodes in the neural network. Alternatively, in the neural network, in the relationship between the nodes based on the link, the initial input node may mean nodes which do not have other input nodes connected through the links. Similarly thereto, the final output node may mean one or more nodes which do not have the output node in the relationship with other nodes among the nodes in the neural network. Further, a hidden node may mean nodes constituting the neural network other than the initial input node and the final output node.

In the neural network according to an exemplary embodiment of the present disclosure, the number of nodes of the input layer may be the same as the number of nodes of the output layer, and the neural network may be a neural network of a type in which the number of nodes decreases and then, increases again from the input layer to the hidden layer. Further, in the neural network according to another exemplary embodiment of the present disclosure, the number of nodes of the input layer may be smaller than the number of nodes of the output layer, and the neural network may be a neural network of a type in which the number of nodes decreases from the input layer to the hidden layer. Further, in the neural network according to yet another exemplary embodiment of the present disclosure, the number of nodes of the input layer may be larger than the number of nodes of the output layer, and the neural network may be a neural network of a type in which the number of nodes increases from the input layer to the hidden layer. The neural network according to still yet another exemplary embodiment of the present disclosure may be a neural network of a type in which the neural networks are combined.

A deep neural network (DNN) may refer to a neural network that includes a plurality of hidden layers in addition to the input and output layers. When the deep neural network is used, the latent structures of data may be determined. That is, latent structures of photos, text, video, voice, and music (e.g., what objects are in the photo, what the content and feelings of the text are, what the content and feelings of the voice are) may be determined. The deep neural network may include a convolutional neural network (CNN), a recurrent neural network (RNN), an auto encoder, generative adversarial networks (GAN), a restricted Boltzmann machine (RBM), a deep belief network (DBN), a Q network, a U network, a Siam network, a Generative Adversarial Network (GAN), and the like. The description of the deep neural network described above is just an example and the present disclosure is not limited thereto.

In an exemplary embodiment of the present disclosure, the network function may include the auto encoder. The auto encoder may be a kind of artificial neural network for outputting output data similar to input data. The auto encoder may include at least one hidden layer and odd hidden layers may be disposed between the input and output layers. The number of nodes in each layer may be reduced from the number of nodes in the input layer to an intermediate layer called a bottleneck layer (encoding), and then expanded symmetrical to reduction to the output layer (symmetrical to the input layer) in the bottleneck layer. The auto encoder may perform non-linear dimensional reduction. The number of input and output layers may correspond to a dimension after preprocessing the input data. The auto encoder structure may have a structure in which the number of nodes in the hidden layer included in the encoder decreases as a distance from the input layer increases. When the number of nodes in the bottleneck layer (a layer having a smallest number of nodes positioned between an encoder and a decoder) is too small, a sufficient amount of information may not be delivered, and as a result, the number of nodes in the bottleneck layer may be maintained to be a specific number or more (e.g., half of the input layers or more).

The neural network may be learned in at least one scheme of supervised learning, unsupervised learning, semi supervised learning, or reinforcement learning. The learning of the neural network may be a process in which the neural network applies knowledge for performing a specific operation to the neural network.

The neural network may be learned in a direction to minimize errors of an output. The learning of the neural network is a process of repeatedly inputting training data into the neural network and calculating the output of the neural network for the training data and the error of a target and back-propagating the errors of the neural network from the output layer of the neural network toward the input layer in a direction to reduce the errors to update the weight of each node of the neural network. In the case of the supervised learning, the training data labeled with a correct answer is used for each training data (i.e., the labeled training data) and in the case of the unsupervised learning, the correct answer may not be labeled in each training data. That is, for example, the training data in the case of the supervised learning related to the data classification may be data in which category is labeled in each training data. The labeled training data is input to the neural network, and the error may be calculated by comparing the output (category) of the neural network with the label of the training data. As another example, in the case of the unsupervised learning related to the data classification, the training data as the input is compared with the output of the neural network to calculate the error. The calculated error is back-propagated in a reverse direction (i.e., a direction from the output layer toward the input layer) in the neural network and connection weights of respective nodes of each layer of the neural network may be updated according to the back propagation. A variation amount of the updated connection weight of each node may be determined according to a learning rate. Calculation of the neural network for the input data and the back-propagation of the error may constitute a learning cycle (epoch). The learning rate may be applied differently according to the number of repetition times of the learning cycle of the neural network. For example, in an initial stage of the learning of the neural network, the neural network ensures a certain level of performance quickly by using a high learning rate, thereby increasing efficiency and uses a low learning rate in a latter stage of the learning, thereby increasing accuracy.

In learning of the neural network, the training data may be generally a subset of actual data (i.e., data to be processed using the learned neural network), and as a result, there may be a learning cycle in which errors for the training data decrease, but the errors for the actual data increase. Overfitting is a phenomenon in which the errors for the actual data increase due to excessive learning of the training data. For example, a phenomenon in which the neural network that learns a cat by showing a yellow cat sees a cat other than the yellow cat and does not recognize the corresponding cat as the cat may be a kind of overfitting. The overfitting may act as a cause which increases the error of the machine learning algorithm. Various optimization methods may be used in order to prevent the overfitting. In order to prevent the overfitting, a method such as increasing the training data, regularization, dropout of omitting a part of the node of the network in the process of learning, utilization of a batch normalization layer, etc., may be applied.

The risk estimation model 300 or the first sub-model 320 according to an embodiment of the present disclosure may borrow at least a part on of a transformer. The transformer may be constituted by an encoder that encodes embedded data and a decoder that decodes the encoded data. The transformer may have a structure that receives a series of data, and outputs a series of data of different types through encoding and decoding steps. In an embodiment, a series of data may be processed in a form which is enabled to be computed by the transformer. A process of processing a series of data in the form which is enabled to be computed by the transformer may include an embedding process. Expressions such as a data token, an embedding vector, embedding token, etc. may refer to data embedded in a form which may be processed by the transformer.

In order for the transformer to encode and decode a series of data, encoders and decoders within the transformer may be processed using an attention algorithm. The attention algorithm may mean an algorithm that obtains a similarity of one or more keys for a given query, reflects the given similarity to a value corresponding to each key, and then calculates an attention value by weighting the values to which the similarity is reflected.

Depending on how the query, key, and value are set, various types of attention algorithms may be classified. For example, if an attention is obtained by setting the query, key, and value all the same, this may mean a self-attention algorithm. In order to process a series of input data in parallel, when a dimension of the embedding vector is reduced and the attention is obtained by obtaining individual attention heads for each divided embedding vector, this may mean a multi-head attention algorithm.

In an embodiment, the transformer may be constituted by modules that perform a plurality of multi-head self-attention algorithms or multi-head encoder-decoder algorithms. In an embodiment, the transformer may also include additional components other than the attention algorithm, such as an embedding layer, a normalization layer, a SoftMax layer, etc. A method for constituting the transformer by using the attention algorithm may include a method disclosed in Vaswani et al., Attention Is All You Need, 2017 NIPS, which is incorporated herein by reference.

The transformer is applied to various data domains such as an embedded natural language, segmented image data, and an audio waveform to convert a series of input data into a series of output data. In order to convert data with various data domains into a series of data that are enabled to be input to the transformer, the transformer may embed the data. The transformer may process additional data expressing a relative positional relationship or phase relationship between a series of input data. Alternatively, the series of input data may be embedded by additionally reflecting vectors expressing relative positional relationships or phase relationships between the input data. In one example, the relative positional relationship between a series of input data may include a word order within the natural language sentence, a relative positional relationship of respective segmented images, a temporal order of segmented audio waveforms, etc., but is not limited thereto. A process of adding information expressing a relative positional relationship or phase relationship between a series of input data may be referred to as positional encoding.

In an embodiment of the present disclosure, the second sub-model 330 estimates the heart age from the acquired ECG data 310 and compares the estimated heart age with an actual age of a subject the atrial fibrillation or the atrial flutter to output the risk for at least one of the atrial fibrillation or the atrial flutter.

In an embodiment, the second sub-model 330 may estimate the heart age to estimate the risk using a difference from the actual age of the current subject. For example, the second sub-model 330 may estimate the heart age from the ECG data in which no atrial fibrillation or atrial flutter occurs by utilizing a machine learning algorithm that learns a dataset including the ECG data and the age of the subject at the time of measurement, or a rule-based heart age estimation method using the ECG data as an input. The second sub-model 330 is an algorithm that estimates the risk of the atrial fibrillation or flutter from the difference between the actual age of the subject and the heart age. For example, it may be determined that the larger the difference between the actual age and the estimated heart age, the higher the risk in the ECG data that is the basis for calculating the heart age.

In an embodiment of the present disclosure, the third sub-model 340 may include a model that changes a first threshold used in the first model 200A that determines whether at least one of the atrial fibrillation or the atrial flutter is found in the acquired ECG data 310 to a second threshold. In an embodiment, the third sub-model 340 may output the risk for at least one of the atrial fibrillation and the atrial flutter from the acquired ECG data 310. Here, the second threshold may be lower than the first threshold. That is, since the third sub-model 340 has a threshold lower than that of the first model 200A for determining whether the atrial fibrillation or atrial flutter is found from the ECG data, the input ECG data is a value lower than the threshold used in the first model 200A, so the ECG data is classified into the normal ECG data in the first model 200A, but when the input ECG data has a value higher than a threshold lower therethan, the ECG data may be determined as the normal ECG data with the high-risk in the third sub-model 340.

For example, the third sub-model 340 may estimate the risk of the input normal ECG data using a logit value or a probability value of a deep learning model that diagnoses the atrial fibrillation or atrial flutter. The third sub-model 340 may utilize the first model 200A that confirms whether the atrial fibrillation or atrial flutter occurs in the present disclosure. As an example, the first model 200A may receive the ECG data and outputs a probability value of the atrial fibrillation or atrial flutter, and at this time, when the probability value exceeds a predetermined threshold, it may be diagnosed that the atrial fibrillation or atrial flutter is present in the input ECG data. Even if the logit or probability value which the first model 200A outputs by receiving the ECG data does not exceed the first threshold set for the first model 200A, by applying the second threshold that is smaller than the first threshold, it may be output whether the ECG data classified as normal falls into the risk group.

In an embodiment of the present disclosure, a fourth sub-model 350 may be generated based on analyzing ECG characteristic indicators including at least one of a PR-interval, an RR-interval, QRS duration, a QT interval, or a QT-corrected interval for high-risk group ECG data and low-risk group ECG data. The fourth sub-model 350 may output the risk for at least one of the atrial fibrillation or the atrial flutter from the acquired ECG data 310. For example, the fourth sub-model 350 may determine ECG characteristics indicators of the ECG data having the high-risk among normal ECG data by analyzing the ECG characteristic indicators such as the PR-interval, the RR-interval, the QRS duration, the QT interval, and/or the QT-corrected interval of the normal ECG data represented as the risk group in the outputs of other models 320, 330, and 340. Using the determined ECG specific indicators, the fourth sub-model 350 may estimate the risk of the input normal ECG data 310.

FIG. 4 illustrates an exemplary algorithm for building training data for electrocardiogram data according to an embodiment of the present disclosure.

In an embodiment, FIG. 4 illustrates an exemplary algorithm for building the training data of the second model 200B. In an embodiment, FIG. 4 illustrates an exemplary algorithm for building the training data of the first sub-model 320.

In FIG. 4, examples are illustrated in which ECG data acquired for respective cases (e.g., Case1, Case2, Case3, and Case4) are aligned according to the time. As an example, one of the cases here may correspond to one subject.

ECG data represented as AFIB/AF ECG in FIG. 4 may mean the ECG data in which the atrial fibrillation or atrial flutter is found. ECG data represented as Non-AFIB/AF ECG in FIG. 4 may mean the normal ECG data in which the atrial fibrillation or atrial flutter is not found.

In FIG. 4, a plurality of bars (e.g., 410, 410a, 410b, 410c...) are illustrated. Each of these bars may mean each measured or acquired ECG data. As an example, one bar may correspond to ECG data measured once. The plurality of bars illustrated in FIG. 4 may be displayed differently depending on whether the atrial fibrillation or atrial flutter is found. These different displays may be displayed as either AFIB/AF ECG or Non-AFIB/AF ECG.

In FIG. 4, the risk group ECG or non-risk group ECG displayed in a rectangular shape indicates that the risk group ECG or non-risk group ECG is used as the training data. The rectangular shape corresponding to the risk group ECG may indicate that the risk group ECG is labeled with a class corresponding to the risk group or high-risk group, and the rectangular shape corresponding to the non-risk group ECG may indicate that the non-risk group ECG is labeled with a class corresponding to the non-risk group or low-risk group.

In an embodiment of the present disclosure, the training data may be generated using ECG data corresponding to each bar illustrated in FIG. 4. As an example, the ECG data corresponding to each bar illustrated in FIG. 4 may be classified into AFIB/AF ECG or Non-AFIB/AF ECG based on the output of the first model 200A. As another example, ECG data corresponding to each bar illustrated in FIG. 4 may be generated using any technique for determining whether the atrial fibrillation or atrial flutter occurs in the ECG data.

In an embodiment of the present disclosure, the training data may be generated by a step of distinguishing the normal ECG data in which no event related to atrial fibrillation or atrial flutter occurs in a dataset containing a plurality of ECG data acquired for the first subject, and abnormal ECG data in which the event occurs, and temporarily sorting the normal ECG data and the abnormal ECG data, and a step of generating training data to which a label is allocated, which corresponds to a first class representing the high-risk group or a second class representing the low-risk group with respect to the normal ECG data based on time information corresponding to each of the distinguished normal ECG data and abnormal ECG data.

The step of generating such training data may include a step of determining, when the abnormal ECG data does not exist in a plurality of ECG data acquired for the first subject, a first time point corresponding to latest ECG data among the plurality of ECG data, and a step of generating training data in which at least one ECG data acquired at time points prior to a first predetermined time period from the first time point is labeled with the second class representing the low-risk group.

For example, the computing device 100 may receive a dataset containing a plurality of ECG data per patient and perform preprocessing. The computing device 100 determines whether the ECG data for each patient is the Atrial Fibrillation (AFIB)/Atrial Flutter (AF) ECG, and aligns the ECG data in order of measurement date. The computing device 100 may label the ECG data represented as the Non-AFIB/AF ECG as the high-risk group or a low-risk group (or the risk group or the non-risk group). A reference for labeling the normal ECG data with the risk group will be described below. When the ECG data represented as the AFIB/AF ECG is present within a predetermined threshold time (e.g., one year) before or after a time point of measuring or acquiring specific normal ECG data represented as the non-AFIB/AF ECG, the normal ECG data may be determined as the high-risk group or risk-group ECG data.

In FIG. 4, Case 1 is referenced. Case 1 illustratively illustrates normal ECG data 410 that may be labeled with a high-risk group or risk group. In an embodiment of the present disclosure, normal ECG data in which there is abnormal ECG data including at least one of the atrial fibrillation or the atrial flutter within a predetermined time period from the time point of acquisition of the normal ECG data may be determined as normal ECG data corresponding to the high-risk group or risk group.

In Case 1, a total of 6 ECG data may be acquired during a time period corresponding to reference numeral 400a. The ECG data 410 may mean normal ECG data represented as Non-AFIB/AF ECG, that is, in which the atrial fibrillation or atrial flutter does not occur. There are three abnormal ECG data (AFIB/AF ECG) 410a, 410b, and 410c within a threshold time period (e.g., one year) from the time point of acquisition of the ECG data 410. Accordingly, the ECG data 410 is determined to be the normal ECG data at the time of measurement, but other ECG data 410a, 410b, and 410c of the same subject temporally adjacent to the ECG data 410 are determined as the abnormal ECG data, so the ECG data 410 is the normal ECG data, but may be determined as the high-risk group or risk group ECG data. Accordingly, the ECG data 410 may be labeled with the high-risk group or risk group class. Accordingly, the ECG data 410 may be used as training data labeled with the high-risk group or risk group class.

In FIG. 4, Case 2 is referenced. Case 2 exemplarily describes normal ECG data 430, 430a, and 430b that may be labeled with the low-risk group or non-risk group. In Case 2, a total of six ECG data 420, 420a, 420b, 430, 430a, and 430b may be acquired during a time period corresponding to reference numeral 400b. All of six ECG data 420, 420a, 420b, 430, 430a, and 430b illustrated in Case 2 are confirmed as the normal ECG data (Non-AFIB/AF ECG).

In Case 2, all ECG data 430, 430a, and 430b acquired prior to a time point corresponding to a predetermined time period (e.g., one year) in the past from the time point of acquisition of the last measured ECG data 420 may be labeled with the low-risk group or non-risk group. Accordingly, the ECG data 430, 430a, and 430b may be labeled with the low-risk group or non-risk group class. That is, the ECG data 430, 430a, and 430b may be used as training data labeled with the low-risk group or non-risk group class.

In an embodiment of the present disclosure, an exemplary scheme for generating training data from the normal ECG data is described. A scheme for generating the training data includes an algorithm for generating training data in a situation in which both the normal ECG data and the abnormal ECG data exist in the plurality of ECG data acquired with respect to the first subject. The scheme for generating the training data may include a step of determining a second time point corresponding to first acquired abnormal ECG data among the plurality of ECG data, a step of determining a third time point corresponding to latest acquired ECG data in at least one ECG data acquired at time points prior to a second predetermined time period from the second time point, and a step of generating training data in which at least one ECG data acquired at time points prior to a third predetermined time period from the third time point is labeled with the second class representing the low-risk group. Here, during the predetermined first time period, the predetermined second time period, and/or the predetermined third time period, the same time period or different time periods may be allocated according to an implementation aspect.

In this regard, in FIG. 4, Case 3 is referenced. Case 3 exemplarily illustrates an algorithm for generating the training data in a situation where both the normal ECG data and the abnormal ECG data exist in a plurality of ECG data acquired for a specific subj ect.

In Case 3, a total of nine ECG data 440, 420a, 420b, 440c, 440d, 450, 450a, 460, and 430a may be acquired during a time period corresponding to reference numeral 400c. Some 440, 440b, and 440c of nine ECG data 440, 420a, 420b, 440c, 440d, 450, 450a, 460, and 430a illustrated in Case 3 are confirmed as the abnormal ECG data AFIB/AF ECG, and some others 440a, 440d, 450, 450a, 460, and 460a are confirmed as the normal ECG data non-AFIB/AF ECG.

In the example of Case 3, first AFIB/AF ECG of the subject is ECG data corresponding to reference numeral 440. That is, latest normal ECG data 450 is determined among ECG data 450, 450a, 460, and 460a acquired at a past time point over a predetermined time period (e.g., one year) or more based on the time point of acquisition of the first ECG data 440 in which the atrial fibrillation or atrial flutter is found among the ECG data of the subject. The low-risk group or non-risk group class may be allocated to the ECG data 460 and 460a acquired in the past over a predetermined time period, for example, one year based on the acquisition time point of the determined ECG data 450. Accordingly, the ECG data 460 and 460a may be labeled with the low-risk group or non-risk group class. That is, the ECG data 460 and 460a may be used as training data labeled with the low-risk group or non-risk group class.

Additionally, it is confirmed that abnormal ECG data 440, 440b, and 440c exist within a predetermined time period (e.g., one year) from the acquisition time point of the normal ECG data 440a in Case 3. Accordingly, the normal ECG data 440a may be labeled with the high-risk group or risk group class. That is, the normal ECG data 440a may be used as training data labeled with the high-risk group or risk group class.

Additionally, for the normal ECG data 440d in Case 3, although there is no abnormal ECG data within a predetermined time period (e.g., one year), but a condition for labeling with the non-risk group or low-risk group is not satisfied, so the normal ECG data 440d may not be used as the training data.

Case 4 of FIG. 4 is referenced. Case 4 exemplarily illustrates an algorithm for generating the training data in a situation where both the normal ECG data and the abnormal ECG data exist in a plurality of ECG data acquired for a specific subject.

In Case 4, a total of ten ECG data 470, 470a, 470b, 470c, 470d, 470e, 470f, 470g, 480, and 480a may be acquired during a time period corresponding to reference numeral 400d. Some 470, 470b, 470c, and 470d of ten ECG data 470, 470a, 470b, 470c, 470d, 470e, 470f, 470g, 480, and 480a illustrated in Case 4 are confirmed as the abnormal ECG data AFIB/AF ECG, and some others 470a, 470e, 470f, 470g, 480, and 480a are confirmed as the normal ECG data non-AFIB/AF ECG.

Since abnormal ECG data 470d and/or 470c exists prior to a predetermined time period from the normal ECG data 470e, the normal ECG data 470e may be labeled as the high-risk group or risk group. Further, since abnormal ECG data 470 and/or 470b exists within to a predetermined time period from the normal ECG data 470a, the normal ECG data 470a may be labeled as the high-risk group or risk group. Further, since abnormal ECG data 470 exists within to a predetermined time period from the normal ECG data 470f and 470g, the normal ECG data 470f and 470g may be labeled as the high-risk group or risk group.

In the example of Case 4, normal ECG data that exists prior to a predetermined period of time (e.g., one year) from the latest acquired abnormal ECG data 470 is represented by reference numeral 480. Since another normal ECG data 480a does not exist in the past over one year from the normal ECG data 480, labeling may not be performed for the ECG data 480a as well as the ECG data 480. That is, the ECG data 480a as well as the ECG data 480 correspond to the normal ECG data, but are not used as the training data for the risk estimation model.

As illustrated in FIG. 4, according to an embodiment of the present disclosure, the normal ECG data may also be easily labeled with risk group (high-risk group) and the non-risk group (low-risk group). By learning the labeled normal ECG data based on deep learning, an artificial intelligence model that classifies the normal ECG data into the risk group may be acquired.

The technique according to an embodiment of the present disclosure, it is possible to present the risk that the atrial fibrillation or atrial flutter may occur in the foreseeable future or past by using ECG data in which no event related to atrial fibrillation or atrial flutter occurs.

Therefore, the technique according to an embodiment of the present disclosure may assist health management and treatment of a patient just before the atrial fibrillation or atrial flutter occurs or patients in which it is difficult to measure an event through the ECG data due to intermittent event occurrence in the early stages of atrial fibrillation or atrial flutter.

In addition, the technique according to an embodiment of the present disclosure examines whether an event related to the atrial fibrillation or atrial flutter is found from a single electrocardiogram measurement, and if not found, additionally may quantitatively determine the risk for the atrial fibrillation or atrial flutter, thereby providing accurate information to the user in a more efficient scheme.
In the meantime, according to an embodiment of the present disclosure, a computer readable medium storing a data structure is disclosed.

The data structure may refer to organization, management, and storage of data that enable efficient access and modification of data. The data structure may refer to organization of data for solving a specific problem (for example, data search, data storage, and data modification in the shortest time). The data structure may also be defined with a physical or logical relationship between the data elements designed to support a specific data processing function. A logical relationship between data elements may include a connection relationship between user defined data elements. A physical relationship between data elements may include an actual relationship between the data elements physically stored in a computer readable storage medium (for example, a permanent storage device). In particular, the data structure may include a set of data, a relationship between data, and a function or a command applicable to data. Through the effectively designed data structure, the computing device may perform a calculation while minimally using resources of the computing device. In particular, the computing device may improve efficiency of calculation, reading, insertion, deletion, comparison, exchange, and search through the effectively designed data structure.

The data structure may be divided into a linear data structure and a non-linear data structure according to the form of the data structure. The linear data structure may be the structure in which only one data is connected after one data. The linear data structure may include a list, a stack, a queue, and a deque. The list may mean a series of dataset in which order exists internally. The list may include a linked list. The linked list may have a data structure in which data is connected in a method in which each data has a pointer and is linked in a single line. In the linked list, the pointer may include information about the connection with the next or previous data. The linked list may be expressed as a single linked list, a double linked list, and a circular linked list according to the form. The stack may have a data listing structure with limited access to data. The stack may have a linear data structure that may process (for example, insert or delete) data only at one end of the data structure. The data stored in the stack may have a data structure (Last In First Out, LIFO) in which the later the data enters, the sooner the data comes out. The queue is a data listing structure with limited access to data, and may have a data structure (First In First Out, FIFO) in which the later the data is stored, the later the data comes out, unlike the stack. The deque may have a data structure that may process data at both ends of the data structure.

The non-linear data structure may be the structure in which the plurality of data is connected after one data. The non-linear data structure may include a graph data structure. The graph data structure may be defined with a vertex and an edge, and the edge may include a line connecting two different vertexes. The graph data structure may include a tree data structure. The tree data structure may be the data structure in which a path connecting two different vertexes among the plurality of vertexes included in the tree is one. That is, the tree data structure may be the data structure in which a loop is not formed in the graph data structure.
Throughout the present specification, a calculation model, a nerve network, the network function, and the neural network may be used with the same meaning. Hereinafter, the terms of the calculation model, the nerve network, the network function, and the neural network are unified and described with a neural network. The data structure may include a neural network. Further, the data structure including the neural network may be stored in a computer readable medium. The data structure including the neural network may also include preprocessed data for processing by the neural network, data input to the neural network, a weight of the neural network, a hyper-parameter of the neural network, data obtained from the neural network, an active function associated with each node or layer of the neural network, and a loss function for training of the neural network. The data structure including the neural network may include predetermined configuration elements among the disclosed configurations. That is, the data structure including the neural network may include the entirety or a predetermined combination of pre-processed data for processing by neural network, data input to the neural network, a weight of the neural network, a hyper parameter of the neural network, data obtained from the neural network, an active function associated with each node or layer of the neural network, and a loss function for training the neural network. In addition to the foregoing configurations, the data structure including the neural network may include predetermined other information determining a characteristic of the neural network. Further, the data structure may include all type of data used or generated in a computation process of the neural network, and is not limited to the foregoing matter. The computer readable medium may include a computer readable recording medium and/or a computer readable transmission medium. The neural network may be formed of a set of interconnected calculation units which are generally referred to as "nodes". The "nodes" may also be called "neurons." The neural network consists of one or more nodes.

The data structure may include data input to the neural network. The data structure including the data input to the neural network may be stored in the computer readable medium. The data input to the neural network may include training data input in the training process of the neural network and/or input data input to the training completed neural network. The data input to the neural network may include data that has undergone pre-processing and/or data to be pre-processed. The pre-processing may include a data processing process for inputting data to the neural network. Accordingly, the data structure may include data to be pre-processed and data generated by the pre-processing. The foregoing data structure is merely an example, and the present disclosure is not limited thereto.

The data structure may include a weight of the neural network (in the present specification, weights and parameters may be used with the same meaning), Further, the data structure including the weight of the neural network may be stored in the computer readable medium. The neural network may include a plurality of weights. The weight is variable, and in order for the neural network to perform a desired function, the weight may be varied by a user or an algorithm. For example, when one or more input nodes are connected to one output node by links, respectively, the output node may determine a data value output from the output node based on values input to the input nodes connected to the output node and the weight set in the link corresponding to each of the input nodes. The foregoing data structure is merely an example, and the present disclosure is not limited thereto.

For a non-limited example, the weight may include a weight varied in the neural network training process and/or the weight when the training of the neural network is completed. The weight varied in the neural network training process may include a weight at a time at which a training cycle starts and/or a weight varied during a training cycle. The weight when the training of the neural network is completed may include a weight of the neural network completing the training cycle. Accordingly, the data structure including the weight of the neural network may include the data structure including the weight varied in the neural network training process and/or the weight when the training of the neural network is completed. Accordingly, it is assumed that the weight and/or a combination of the respective weights are included in the data structure including the weight of the neural network. The foregoing data structure is merely an example, and the present disclosure is not limited thereto.

The data structure including the weight of the neural network may be stored in the computer readable storage medium (for example, a memory and a hard disk) after undergoing a serialization process. The serialization may be the process of storing the data structure in the same or different computing devices and converting the data structure into a form that may be reconstructed and used later. The computing device may serialize the data structure and transceive the data through a network. The serialized data structure including the weight of the neural network may be reconstructed in the same or different computing devices through deserialization. The data structure including the weight of the neural network is not limited to the serialization. Further, the data structure including the weight of the neural network may include a data structure (for example, in the non-linear data structure, B-Tree, Trie, m-way search tree, AVL tree, and Red-Black Tree) for improving efficiency of the calculation while minimally using the resources of the computing device. The foregoing matter is merely an example, and the present disclosure is not limited thereto.

The data structure may include a hyper-parameter of the neural network. The data structure including the hyper-parameter of the neural network may be stored in the computer readable medium. The hyper-parameter may be a variable varied by a user. The hyper-parameter may include, for example, a learning rate, a cost function, the number of times of repetition of the training cycle, weight initialization (for example, setting of a range of a weight value to be weight-initialized), and the number of hidden units (for example, the number of hidden layers and the number of nodes of the hidden layer). The foregoing data structure is merely an example, and the present disclosure is not limited thereto.

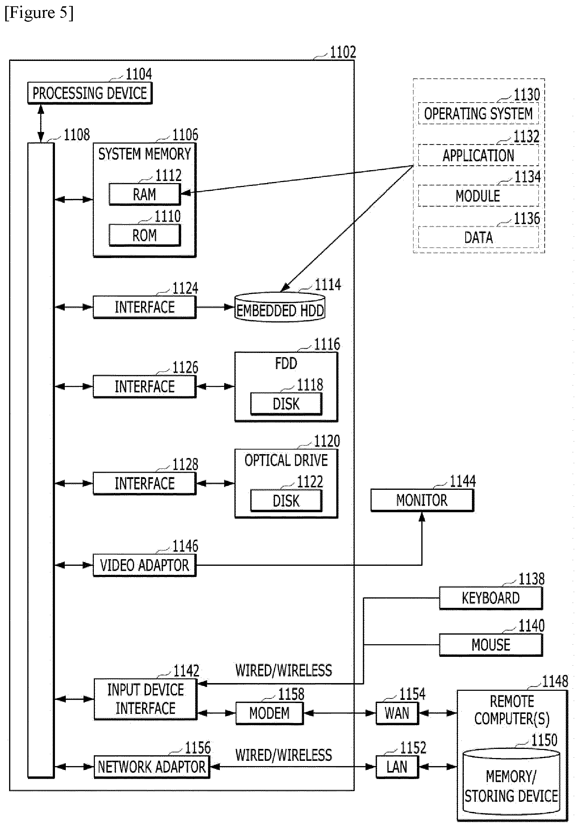

FIG. 5 is a simple and general schematic diagram illustrating an example of a computing environment in which the embodiments of the present disclosure are implementable.

The present disclosure has been described as being generally implementable by the computing device, but those skilled in the art will appreciate well that the present disclosure is combined with computer executable commands and/or other program modules executable in one or more computers and/or be implemented by a combination of hardware and software.

In general, a program module includes a routine, a program, a component, a data structure, and the like performing a specific task or implementing a specific abstract data form. Further, those skilled in the art will well appreciate that the method of the present disclosure may be carried out by a personal computer, a hand-held computing device, a microprocessor-based or programmable home appliance (each of which may be connected with one or more relevant devices and be operated), and other computer system configurations, as well as a single-processor or multiprocessor computer system, a mini computer, and a main frame computer.

The embodiments of the present disclosure may be carried out in a distribution computing environment, in which certain tasks are performed by remote processing devices connected through a communication network. In the distribution computing environment, a program module may be located in both a local memory storage device and a remote memory storage device.

The computer generally includes various computer readable media. The computer accessible medium may be any type of computer readable medium, and the computer readable medium includes volatile and non-volatile media, transitory and non-transitory media, and portable and non-portable media. As a non-limited example, the computer readable medium may include a computer readable storage medium and a computer readable transport medium. The computer readable storage medium includes volatile and non-volatile media, transitory and non-transitory media, and portable and non-portable media constructed by a predetermined method or technology, which stores information, such as a computer readable command, a data structure, a program module, or other data. The computer readable storage medium includes a RAM, a Read Only Memory (ROM), an Electrically Erasable and Programmable ROM (EEPROM), a flash memory, or other memory technologies, a Compact Disc (CD)-ROM, a Digital Video Disk (DVD), or other optical disk storage devices, a magnetic cassette, a magnetic tape, a magnetic disk storage device, or other magnetic storage device, or other predetermined media, which are accessible by a computer and are used for storing desired information, but is not limited thereto.

The computer readable transport medium generally implements a computer readable command, a data structure, a program module, or other data in a modulated data signal, such as a carrier wave or other transport mechanisms, and includes all of the information transport media. The modulated data signal means a signal, of which one or more of the characteristics are set or changed so as to encode information within the signal. As a non-limited example, the computer readable transport medium includes a wired medium, such as a wired network or a direct-wired connection, and a wireless medium, such as sound, Radio Frequency (RF), infrared rays, and other wireless media. A combination of the predetermined media among the foregoing media is also included in a range of the computer readable transport medium.

An illustrative environment 1100 including a computer 1102 and implementing several aspects of the present disclosure is illustrated, and the computer 1102 includes a processing device 1104, a system memory 1106, and a system bus 1108. The system bus 1108 connects system components including the system memory 1106 (not limited) to the processing device 1104. The processing device 1104 may be a predetermined processor among various commonly used processors. A dual processor and other multiprocessor architectures may also be used as the processing device 1104.

The system bus 1108 may be a predetermined one among several types of bus structure, which may be additionally connectable to a local bus using a predetermined one among a memory bus, a peripheral device bus, and various common bus architectures. The system memory 1106 includes a ROM 1110, and a RAM 1112. A basic input/output system (BIOS) is stored in a non-volatile memory 1110, such as a ROM, an EPROM, and an EEPROM, and the BIOS includes a basic routing helping a transport of information among the constituent elements within the computer 1102 at a time, such as starting. The RAM 1112 may also include a high-rate RAM, such as a static RAM, for caching data.

The computer 1102 also includes an embedded hard disk drive (HDD) 1114 (for example, enhanced integrated drive electronics (EIDE) and serial advanced technology attachment (SATA)) - the embedded HDD 1114 being configured for exterior mounted usage within a proper chassis (not illustrated) - a magnetic floppy disk drive (FDD) 1116 (for example, which is for reading data from a portable diskette 1118 or recording data in the portable diskette 1118), and an optical disk drive 1120 (for example, which is for reading a CD-ROM disk 1122, or reading data from other high-capacity optical media, such as a DVD, or recording data in the high-capacity optical media). A hard disk drive 1114, a magnetic disk drive 1116, and an optical disk drive 1120 may be connected to a system bus 1108 by a hard disk drive interface 1124, a magnetic disk drive interface 1126, and an optical drive interface 1128, respectively. An interface 1124 for implementing an outer mounted drive includes, for example, at least one of or both a universal serial bus (USB) and the Institute of Electrical and Electronics Engineers (IEEE) 1394 interface technology.

The drives and the computer readable media associated with the drives provide non-volatile storage of data, data structures, computer executable commands, and the like. In the case of the computer 1102, the drive and the medium correspond to the storage of random data in an appropriate digital form. In the description of the computer readable media, the HDD, the portable magnetic disk, and the portable optical media, such as a CD, or a DVD, are mentioned, but those skilled in the art will well appreciate that other types of computer readable media, such as a zip drive, a magnetic cassette, a flash memory card, and a cartridge, may also be used in the illustrative operation environment, and the predetermined medium may include computer executable commands for performing the methods of the present disclosure.

A plurality of program modules including an operation system 1130, one or more application programs 1132, other program modules 1134, and program data 1136 may be stored in the drive and the RAM 1112. An entirety or a part of the operation system, the application, the module, and/or data may also be cached in the RAM 1112. It will be well appreciated that the present disclosure may be implemented by several commercially usable operation systems or a combination of operation systems.

A user may input a command and information to the computer 1102 through one or more wired/wireless input devices, for example, a keyboard 1138 and a pointing device, such as a mouse 1140. Other input devices (not illustrated) may be a microphone, an IR remote controller, a joystick, a game pad, a stylus pen, a touch screen, and the like. The foregoing and other input devices are frequently connected to the processing device 1104 through an input device interface 1142 connected to the system bus 1108, but may be connected by other interfaces, such as a parallel port, an IEEE 1394 serial port, a game port, a USB port, an IR interface, and other interfaces.

A monitor 1144 or other types of display devices are also connected to the system bus 1108 through an interface, such as a video adaptor 1146. In addition to the monitor 1144, the computer generally includes other peripheral output devices (not illustrated), such as a speaker and a printer.

The computer 1102 may be operated in a networked environment by using a logical connection to one or more remote computers, such as remote computer(s) 1148, through wired and/or wireless communication. The remote computer(s) 1148 may be a work station, a computing device computer, a router, a personal computer, a portable computer, a microprocessor-based entertainment device, a peer device, and other general network nodes, and generally includes some or an entirety of the constituent elements described for the computer 1102, but only a memory storage device 1150 is illustrated for simplicity. The illustrated logical connection includes a wired/wireless connection to a local area network (LAN) 1152 and/or a larger network, for example, a wide area network (WAN) 1154. The LAN and WAN networking environments are general in an office and a company, and make an enterprise-wide computer network, such as an Intranet, easy, and all of the LAN and WAN networking environments may be connected to a worldwide computer network, for example, the Internet.

When the computer 1102 is used in the LAN networking environment, the computer 1102 is connected to the local network 1152 through a wired and/or wireless communication network interface or an adaptor 1156. The adaptor 1156 may make wired or wireless communication to the LAN 1152 easy, and the LAN 1152 also includes a wireless access point installed therein for the communication with the wireless adaptor 1156. When the computer 1102 is used in the WAN networking environment, the computer 1102 may include a modem 1158, is connected to a communication computing device on a WAN 1154, or includes other means setting communication through the WAN 1154 via the Internet. The modem 1158, which may be an embedded or outer-mounted and wired or wireless device, is connected to the system bus 1108 through a serial port interface 1142. In the networked environment, the program modules described for the computer 1102 or some of the program modules may be stored in a remote memory/storage device 1150. The illustrated network connection is illustrative, and those skilled in the art will appreciate well that other means setting a communication link between the computers may be used.

The computer 1102 performs an operation of communicating with a predetermined wireless device or entity, for example, a printer, a scanner, a desktop and/or portable computer, a portable data assistant (PDA), a communication satellite, predetermined equipment or place related to a wirelessly detectable tag, and a telephone, which is disposed by wireless communication and is operated. The operation includes a wireless fidelity (Wi-Fi) and Bluetooth wireless technology at least. Accordingly, the communication may have a pre-defined structure, such as a network in the related art, or may be simply ad hoc communication between at least two devices.

The Wi-Fi enables a connection to the Internet and the like even without a wire. The Wi-Fi is a wireless technology, such as a cellular phone, which enables the device, for example, the computer, to transmit and receive data indoors and outdoors, that is, in any place within a communication range of a base station. A Wi-Fi network uses a wireless technology, which is called IEEE 802.11 (a, b, g, etc.) for providing a safe, reliable, and high-rate wireless connection. The Wi-Fi may be used for connecting the computer to the computer, the Internet, and the wired network (IEEE 802.3 or Ethernet is used). The Wi-Fi network may be operated at, for example, a data rate of 11 Mbps (802.11a) or 54 Mbps (802.11b) in an unauthorized 2.4 and 5 GHz wireless band, or may be operated in a product including both bands (dual bands).

Those skilled in the art may appreciate that information and signals may be expressed by using predetermined various different technologies and techniques. For example, data, indications, commands, information, signals, bits, symbols, and chips referable in the foregoing description may be expressed with voltages, currents, electromagnetic waves, magnetic fields or particles, optical fields or particles, or a predetermined combination thereof.

Those skilled in the art will appreciate that the various illustrative logical blocks, modules, processors, means, circuits, and algorithm operations described in relationship to the embodiments disclosed herein may be implemented by electronic hardware (for convenience, called "software" herein), various forms of program or design code, or a combination thereof. In order to clearly describe compatibility of the hardware and the software, various illustrative components, blocks, modules, circuits, and operations are generally illustrated above in relation to the functions of the hardware and the software. Whether the function is implemented as hardware or software depends on design limits given to a specific application or an entire system. Those skilled in the art may perform the function described by various schemes for each specific application, but it shall not be construed that the determinations of the performance depart from the scope of the present disclosure.

Various embodiments presented herein may be implemented by a method, a device, or a manufactured article using a standard programming and/or engineering technology. A term "manufactured article" includes a computer program, a carrier, or a medium accessible from a predetermined computer-readable storage device. For example, the computer-readable storage medium includes a magnetic storage device (for example, a hard disk, a floppy disk, and a magnetic strip), an optical disk (for example, a CD and a DVD), a smart card, and a flash memory device (for example, an EEPROM, a card, a stick, and a key drive), but is not limited thereto. Further, various storage media presented herein include one or more devices and/or other machine-readable media for storing information.

It shall be understood that a specific order or a hierarchical structure of the operations included in the presented processes is an example of illustrative accesses. It shall be understood that a specific order or a hierarchical structure of the operations included in the processes may be rearranged within the scope of the present disclosure based on design priorities. The accompanying method claims provide various operations of elements in a sample order, but it does not mean that the claims are limited to the presented specific order or hierarchical structure.

The description of the presented embodiments is provided so as for those skilled in the art to use or carry out the present disclosure. Various modifications of the embodiments may be apparent to those skilled in the art, and general principles defined herein may be applied to other embodiments without departing from the scope of the present disclosure. Accordingly, the present disclosure is not limited to the embodiments suggested herein, and shall be interpreted within the broadest meaning range consistent to the principles and new characteristics presented herein.

## Claims

1. A method for analyzing electrocardiogram (ECG) data performed by a computing device, the method comprising:
determining, by using a first model having ECG data acquired by ECG measurement as an input, whether an event related to at least one of atrial fibrillation or atrial flutter is found in the acquired ECG data;
generating a first diagnosis result for the acquired ECG data when the event is found in the acquired ECG data; and
generating a second diagnosis result for estimating a risk for at least one of the atrial fibrillation or the atrial flutter by using a second model having the acquired ECG data as an input when the event is not found in the acquired ECG data.

2. The method of claim 1, wherein the first model and the second model are trained based on different training data, and trained based on different training methods.

3. The method of claim 1, wherein the first model includes at least one of:
an artificial intelligence-based model trained based on ECG data including at least one of the atrial fibrillation or the atrial flutter, or
a rule-based model generated based on feature information for at least one of the atrial fibrillation or the atrial flutter.

4. The method of claim 1, wherein the second model is an artificial intelligence-based model trained based on normal ECG data in which the event does not occur.

5. The method of claim 4, wherein the second model is an artificial intelligence-based model trained based on first training data in which first normal ECG data is labeled with a first class representing a high-risk group, and second training data in which second normal ECG data is labeled with a second class representing a low-risk group, and
wherein the normal ECG data in which abnormal ECG data including at least one of the atrial fibrillation or the atrial flutter within a predetermined time period from an acquisition time point of the normal ECG data exists is determined as the first normal ECG data.

6. The method of claim 1, wherein the second model is pre-trained based on:
distinguishing normal ECG data in which the event does not occur and abnormal ECG data in which the event occurs in a dataset with a plurality of ECG data acquired for a first subject, and temporally aligning the distinguished normal ECG data and abnormal ECG data, and
generating, based on time information corresponding to each of the distinguished normal ECG data and abnormal ECG data, training data to which a label corresponding to a first class representing a high-risk group or a second class representing a low-risk group is allocated with respect to the normal ECG data.

7. The method of claim 6, wherein the generating of the training data includes:
generating, additionally based on at least one of prescription record information or surgery history information allocated to the distinguished normal ECG data, the training data to which the label corresponding to the first class representing the high-risk group or the second class representing the low-risk group is allocated with respect to the normal ECG data.

8. The method of claim 6, wherein the generating of the training data includes:
determining a first time point corresponding to latest ECG data among the plurality of ECG data when the abnormal ECG data does not exist in the plurality of ECG data acquired for the first subject, and
generating the training data in which at least one ECG data acquired at time points prior to a first predetermined time period from the first time point is labeled with the second class representing the low-risk group.

9. The method of claim 6, wherein the generating of the training data includes:
determining a second time point corresponding to first abnormal ECG data among the plurality of ECG data when the normal ECG data and the abnormal ECG data exist in the plurality of ECG data acquired for the first subject,
determining a third time point corresponding latest ECG data in at least one ECG data acquired at time points prior to a second predetermined time period from the second time point, and
generating the training data in which at least one ECG data acquired at time points prior to a third predetermined time period from the third time point is labeled with the second class representing the low-risk group.

10. The method of claim 1, wherein the second model analyzes a structural change in an atria from normal ECG data in which the event does not occur to output the risk including a possibility of past or future occurrence of the atrial fibrillation or the atrial flutter.

11. The method of claim 1, wherein the first diagnosis result includes a result representing that there is a possibility that at least one of the atrial fibrillation or the atrial flutter will exist with respect to the acquired ECG data, and
wherein the second diagnosis result includes a result representing that at least one of the atrial fibrillation or the atrial flutter does not exist with respect to the acquired ECG data, but there is a possibility of past or future occurrence of at least one of the atrial fibrillation or the atrial flutter with respect to the acquired ECG data.

12. The method of claim 1, wherein the second model includes a deep learned based first sub-model which is trained based on a plurality of normal ECG data aligned in order of acquisition time and outputs the risk for at least one of the atrial fibrillation or the atrial flutter in response to the acquired ECG data.

13. The method of claim 12, wherein the second model further includes a second sub-model which estimates the heart age from the acquired ECG data and compares the estimated heart age with an actual age of a subject to output the risk for at least one of the atrial fibrillation or the atrial flutter.

14. The method of claim 12, wherein the second model further includes a third sub-model which changes a first threshold used in the first model which determines whether at least one of the atrial fibrillation or the atrial flutter is found in the acquired ECG data to a second threshold, where the second threshold is lower than the first threshold, and
wherein the third sub-model outputs the risk for at least one of the atrial fibrillation or the atrial flutter from the acquired ECG data.

15. The method of claim 12, wherein the second model further includes a fourth sub-model generated based on analyzing ECG characteristic indicators including at least one of a PR-interval, an RR-interval, QRS duration, a QT interval, or a QT-corrected interval for high-risk group ECG data and low-risk group ECG data, and
wherein the fourth sub-model outputs the risk for at least one of the atrial fibrillation or the atrial flutter from the acquired ECG data.

16. A computing device for analyzing electrocardiogram (ECG) data, comprising:
at least one processor; and
a memory,
wherein the at least one processor:
determines, by using a first model having ECG data acquired by ECG measurement as an input, whether an event related to at least one of atrial fibrillation or atrial flutter is found in the acquired ECG data,
generates a first diagnosis result for the acquired ECG data when the event is found in the acquired ECG data, and
generates a second diagnosis result for estimating a risk for at least one of the atrial fibrillation or the atrial flutter by using a second model having the acquired ECG data as an input when the event is not found in the acquired ECG data.

17. A computer program stored in a computer readable storage medium,
wherein when the computer program is executed by at least one processor, the computer program allows the at least one processor to perform a method for analyzing electrocardiogram (ECG) data, and
the method includes:
determining, by using a first model having ECG data acquired by ECG measurement as an input, whether an event related to at least one of atrial fibrillation or atrial flutter is found in the acquired ECG data,
generating a first diagnosis result for the acquired ECG data when the event is found in the acquired ECG data; and
generating a second diagnosis result for estimating a risk for at least one of the atrial fibrillation or the atrial flutter by using a second model having the acquired ECG data as an input when the event is not found in the acquired ECG data.
